# EUROPEAN PATENT APPLICATION

(11) **EP 1 538 217 A1**
(43) Date of publication of application: **08.06.2005**
(21) Application number: 03795355.1
(22) Date of filing: 10.09.2003
(51) Int. Cl.: C12Q 1/02, C12Q 1/66, C07K 14/72, G01N 33/15, G01N 33/50, A61K 45/00, A61P 3/10

(54) **METHOD OF SCREENING INSULIN CONTENT ENHANCER**

(30) Priority: 11.09.2002 JP 2002265622; 04.03.2003 JP 2003056813
(71) Applicant: YAMANOUCHI PHARMACEUTICAL CO., LTD., Tokyo 103 (JP)
(72) Inventor: OHISHI, Takahide, Tsukuba-shi, Ibaraki 305-858 (JP); KOIZUMI, Tomonobu, Tsukuba-shi, Ibaraki 305-8585 (JP)
(74) Representative: HOFFMANN EITLE
(86) International application number: PCT/JP2003/011548
(87) International publication number: WO 2004/024943

(57) **Abstract**

A screening tool for an agent for promoting insulin production and/or an agent for increasing insulin content, wherein the tool is a G protein-coupled receptor exhibiting an activity of promoting insulin production by activation, or a cell expressing the polypeptide, is disclosed. Further, a method for screening an agent for promoting insulin production and/or an agent for increasing insulin content, comprising the steps of bringing the cell or a cell membrane thereof into contact with a substance to be tested, and analyzing whether or not the polypeptide is activated, is disclosed.

The screening tool and the screening method are useful in screening a substance which increases insulin content and prevents and/or treats diabetes.

Furthermore, a novel agent for increasing insulin content comprising as an active ingredient a substance obtained by the screening, is disclosed.

SEQUENCE LISTING

<110> Yamanouchi Pharmaceutical Co., Ltd.

<120> Screening method of agents for increasing insulin content

<130> Y0352PCT-698

<150> JP 2002-265622
<151> 2002-09-11

<150> JP 2003-056813
<151> 2003-03-04

<160> 7

<210> 1
<211> 1008
<212> DNA
<213> Homo sapiens

<220>
<223> Inventor: Ohishi, Takahide; Koizumi, Tomonobu

<220>
<221> CDS
<222> (1)..(1008)

<400> 1
atg gaa tca tct ttc tca ttt gga gtg atc ctt gct gtc ctg gcc tcc 48
Met Glu Ser Ser Phe Ser Phe Gly Val Ile Leu Ala Val Leu Ala Ser
1               5                   10                  15

ctc atc att gct act aac aca cta gtg gct gtg gct gtg ctg ctg ttg 96
Leu Ile Ile Ala Thr Asn Thr Leu Val Ala Val Ala Val Leu Leu Leu
            20                  25                  30

atc cac aag aat gat ggt gtc agt ctc tgc ttc acc ttg aat ctg gct 144
Ile His Lys Asn Asp Gly Val Ser Leu Cys Phe Thr Leu Asn Leu Ala
        35                  40                  45

gtg gct gac acc ttg att ggt gtg gcc atc tct ggc cta ctc aca gac 192
Val Ala Asp Thr Leu Ile Gly Val Ala Ile Ser Gly Leu Leu Thr Asp
    50                  55                  60

cag ctc tcc agc cct tct cgg ccc aca cag aag acc ctg tgc agc ctg 240

Gln Leu Ser Ser Pro Ser Arg Pro Thr Gln Lys Thr Leu Cys Ser Leu
65                  70                  75                  80

cgg atg gca ttt gtc act tcc tcc gca gct gcc tct gtc ctc acg gtc     288
Arg Met Ala Phe Val Thr Ser Ser Ala Ala Ala Ser Val Leu Thr Val
                85                  90                  95

atg ctg atc acc ttt gac agg tac ctt gcc atc aag cag ccc ttc cgc     336
Met Leu Ile Thr Phe Asp Arg Tyr Leu Ala Ile Lys Gln Pro Phe Arg
            100                 105                 110

tac ttg aag atc atg agt ggg ttc gtg gcc ggg gcc tgc att gcc ggg     384
Tyr Leu Lys Ile Met Ser Gly Phe Val Ala Gly Ala Cys Ile Ala Gly
        115                 120                 125

ctg tgg tta gtg tct tac ctc att ggc ttc ctc cca ctc gga atc ccc     432
Leu Trp Leu Val Ser Tyr Leu Ile Gly Phe Leu Pro Leu Gly Ile Pro
    130                 135                 140

atg ttc cag cag act gcc tac aaa ggg cag tgc agc ttc ttt gct gta     480
Met Phe Gln Gln Thr Ala Tyr Lys Gly Gln Cys Ser Phe Phe Ala Val
145                 150                 155                 160

ttt cac cct cac ttc gtg ctg acc ctc tcc tgc gtt ggc ttc ttc cca     528
Phe His Pro His Phe Val Leu Thr Leu Ser Cys Val Gly Phe Phe Pro
                165                 170                 175

gcc atg ctc ctc ttt gtc ttc ttc tac tgc gac atg ctc aag att gcc     576
Ala Met Leu Leu Phe Val Phe Phe Tyr Cys Asp Met Leu Lys Ile Ala
            180                 185                 190

tcc atg cac agc cag cag att cga aag atg gaa cat gca gga gcc atg     624
Ser Met His Ser Gln Gln Ile Arg Lys Met Glu His Ala Gly Ala Met
        195                 200                 205

gct gga ggt tat cga tcc cca cgg act ccc agc gac ttc aaa gct ctc     672
Ala Gly Gly Tyr Arg Ser Pro Arg Thr Pro Ser Asp Phe Lys Ala Leu
    210                 215                 220

cgt act gtg tct gtt ctc att ggg agc ttt gct cta tcc tgg acc ccc     720
Arg Thr Val Ser Val Leu Ile Gly Ser Phe Ala Leu Ser Trp Thr Pro
225                 230                 235                 240

ttc ctt atc act ggc att gtg cag gtg gcc tgc cag gag tgt cac ctc     768
Phe Leu Ile Thr Gly Ile Val Gln Val Ala Cys Gln Glu Cys His Leu
                245                 250                 255

tac cta gtg ctg gaa cgg tac ctg tgg ctg ctc ggc gtg ggc aac tcc    816
Tyr Leu Val Leu Glu Arg Tyr Leu Trp Leu Leu Gly Val Gly Asn Ser
            260                 265                 270

ctg ctc aac cca ctc atc tat gcc tat tgg cag aag gag gtg cga ctg    864
Leu Leu Asn Pro Leu Ile Tyr Ala Tyr Trp Gln Lys Glu Val Arg Leu
        275                 280                 285

cag ctc tac cac atg gcc cta gga gtg aag aag gtg ctc acc tca ttc    912
Gln Leu Tyr His Met Ala Leu Gly Val Lys Lys Val Leu Thr Ser Phe
    290                 295                 300

ctc ctc ttt ctc tcg gcc agg aat tgt ggc cca gag agg ccc agg gaa    960
Leu Leu Phe Leu Ser Ala Arg Asn Cys Gly Pro Glu Arg Pro Arg Glu
305                 310                 315                 320

agt tcc tgt cac atc gtc act atc tcc agc tca gag ttt gat ggc taa    1008
Ser Ser Cys His Ile Val Thr Ile Ser Ser Ser Glu Phe Asp Gly
                325                 330                 335

<210> 2
<211> 335
<212> PRT
<213> Homo sapiens

<400> 2
Met Glu Ser Ser Phe Ser Phe Gly Val Ile Leu Ala Val Leu Ala Ser
1               5                   10                  15
Leu Ile Ile Ala Thr Asn Thr Leu Val Ala Val Ala Val Leu Leu Leu
            20                  25                  30
Ile His Lys Asn Asp Gly Val Ser Leu Cys Phe Thr Leu Asn Leu Ala
        35                  40                  45
Val Ala Asp Thr Leu Ile Gly Val Ala Ile Ser Gly Leu Leu Thr Asp
    50                  55                  60
Gln Leu Ser Ser Pro Ser Arg Pro Thr Gln Lys Thr Leu Cys Ser Leu
65                  70                  75                  80
Arg Met Ala Phe Val Thr Ser Ser Ala Ala Ala Ser Val Leu Thr Val
                85                  90                  95
Met Leu Ile Thr Phe Asp Arg Tyr Leu Ala Ile Lys Gln Pro Phe Arg
            100                 105                 110
Tyr Leu Lys Ile Met Ser Gly Phe Val Ala Gly Ala Cys Ile Ala Gly
        115                 120                 125
Leu Trp Leu Val Ser Tyr Leu Ile Gly Phe Leu Pro Leu Gly Ile Pro
    130                 135                 140

Met Phe Gln Gln Thr Ala Tyr Lys Gly Gln Cys Ser Phe Phe Ala Val
145                 150                 155                 160
Phe His Pro His Phe Val Leu Thr Leu Ser Cys Val Gly Phe Phe Pro
                165                 170                 175
Ala Met Leu Leu Phe Val Phe Phe Tyr Cys Asp Met Leu Lys Ile Ala
            180                 185                 190
Ser Met His Ser Gln Gln Ile Arg Lys Met Glu His Ala Gly Ala Met
        195                 200                 205
Ala Gly Gly Tyr Arg Ser Pro Arg Thr Pro Ser Asp Phe Lys Ala Leu
    210                 215                 220
Arg Thr Val Ser Val Leu Ile Gly Ser Phe Ala Leu Ser Trp Thr Pro
225                 230                 235                 240
Phe Leu Ile Thr Gly Ile Val Gln Val Ala Cys Gln Glu Cys His Leu
                245                 250                 255
Tyr Leu Val Leu Glu Arg Tyr Leu Trp Leu Leu Gly Val Gly Asn Ser
            260                 265                 270
Leu Leu Asn Pro Leu Ile Tyr Ala Tyr Trp Gln Lys Glu Val Arg Leu
        275                 280                 285
Gln Leu Tyr His Met Ala Leu Gly Val Lys Lys Val Leu Thr Ser Phe
    290                 295                 300
Leu Leu Phe Leu Ser Ala Arg Asn Cys Gly Pro Glu Arg Pro Arg Glu
305                 310                 315                 320
Ser Ser Cys His Ile Val Thr Ile Ser Ser Ser Glu Phe Asp Gly
                325                 330                 335

<210>  3
<211>  1008
<212>  DNA
<213>  Rattus sp.

<220>
<221>  CDS
<222>  (1)..(1008)

<400>  3
atg gag tca tct ttc tca ttt gga gtg atc ctt gct gtc ctg acc atc      48
Met Glu Ser Ser Phe Ser Phe Gly Val Ile Leu Ala Val Leu Thr Ile
1               5                   10                  15

ctt atc att gct gtt aat gcg ctg gtg gtt gtg gct atg ctg cta tca      96
Leu Ile Ile Ala Val Asn Ala Leu Val Val Val Ala Met Leu Leu Ser
            20                  25                  30

atc tac aag aat gat ggt gtt ggc ctt tgc ttc acc tta aat ctg gcc      144
Ile Tyr Lys Asn Asp Gly Val Gly Leu Cys Phe Thr Leu Asn Leu Ala

35                  40                  45

gtg gct gat acc ttg att ggc gtg gct att tct ggg cta gtt aca gac     192
Val Ala Asp Thr Leu Ile Gly Val Ala Ile Ser Gly Leu Val Thr Asp
    50                  55                  60

cag ctc tcc agc tct gct cag cac aca cag aag acc ttg tgt agc ctt     240
Gln Leu Ser Ser Ser Ala Gln His Thr Gln Lys Thr Leu Cys Ser Leu
65                  70                  75                  80

cgg atg gca ttc gtc act tct tct gca gcc gcc tct gtc ctc acg gtc     288
Arg Met Ala Phe Val Thr Ser Ser Ala Ala Ala Ser Val Leu Thr Val
                85                  90                  95

atg ctg att gcc ttt gac agg tac ctg gcc att aag cag ccc ctc cgt     336
Met Leu Ile Ala Phe Asp Arg Tyr Leu Ala Ile Lys Gln Pro Leu Arg
            100                 105                 110

tac ttc cag atc atg aat ggg ctt gta gcc gga gga tgc att gca ggg     384
Tyr Phe Gln Ile Met Asn Gly Leu Val Ala Gly Gly Cys Ile Ala Gly
        115                 120                 125

ctg tgg ttg ata tct tac ctt atc ggc ttc ctc cca ctt gga gtc tcc     432
Leu Trp Leu Ile Ser Tyr Leu Ile Gly Phe Leu Pro Leu Gly Val Ser
    130                 135                 140

ata ttc cag cag acc acc tac cat ggg ccc tgc acc ttc ttt gct gtg     480
Ile Phe Gln Gln Thr Thr Tyr His Gly Pro Cys Thr Phe Phe Ala Val
145                 150                 155                 160

ttt cac cca agg ttt gtg ctg acc ctc tcc tgt gct ggc ttc ttc cca     528
Phe His Pro Arg Phe Val Leu Thr Leu Ser Cys Ala Gly Phe Phe Pro
                165                 170                 175

gct gtg ctc ctc ttt gtc ttc ttc tac tgt gac atg ctc aag att gcc     576
Ala Val Leu Leu Phe Val Phe Phe Tyr Cys Asp Met Leu Lys Ile Ala
            180                 185                 190

tct gtg cac agc cag cac atc cgg aag atg gaa cat gca gga gcc atg     624
Ser Val His Ser Gln His Ile Arg Lys Met Glu His Ala Gly Ala Met
        195                 200                 205

gtt gga gct tgc cgg ccc cca cgg cct gtc aat gac ttc aag gct gtc     672
Val Gly Ala Cys Arg Pro Pro Arg Pro Val Asn Asp Phe Lys Ala Val
    210                 215                 220

cgg act gta tct gtc ctt att ggg agc ttc acc ctg tcc tgg tct ccg     720
Arg Thr Val Ser Val Leu Ile Gly Ser Phe Thr Leu Ser Trp Ser Pro
225                 230                 235                 240

ttt ctc atc act agc att gtg cag gtg gcc tgc cac aaa tgc tgc ctc     768
Phe Leu Ile Thr Ser Ile Val Gln Val Ala Cys His Lys Cys Cys Leu
                245                 250                 255

tac caa gtg ctg gaa aaa tac ctc tgg ctc ctt gga gtt ggc aac tcc     816
Tyr Gln Val Leu Glu Lys Tyr Leu Trp Leu Leu Gly Val Gly Asn Ser
            260                 265                 270

ctg ctc aac cca ctc atc tat gcc tat tgg cag agg gag gtt cgg cag     864
Leu Leu Asn Pro Leu Ile Tyr Ala Tyr Trp Gln Arg Glu Val Arg Gln
        275                 280                 285

cag ctc tgc cac atg gcc ctg ggg gtg aag aag ttc ttt act tca atc     912
Gln Leu Cys His Met Ala Leu Gly Val Lys Lys Phe Phe Thr Ser Ile
    290                 295                 300

ttc ctc ctt ctc tcg gcc agg aat cgt ggt cca cag agg acc cga gaa     960
Phe Leu Leu Leu Ser Ala Arg Asn Arg Gly Pro Gln Arg Thr Arg Glu
305                 310                 315                 320

agc tcc tat cac atc gtc act atc agc cag ccg gag ctc gat ggc tag     1008
Ser Ser Tyr His Ile Val Thr Ile Ser Gln Pro Glu Leu Asp Gly
                325                 330                 335

<210>  4
<211>  335
<212>  PRT
<213>  Rattus sp.

<400>  4
Met Glu Ser Ser Phe Ser Phe Gly Val Ile Leu Ala Val Leu Thr Ile
  1               5                  10                  15
Leu Ile Ile Ala Val Asn Ala Leu Val Val Val Ala Met Leu Leu Ser
             20                  25                  30
Ile Tyr Lys Asn Asp Gly Val Gly Leu Cys Phe Thr Leu Asn Leu Ala
         35                  40                  45
Val Ala Asp Thr Leu Ile Gly Val Ala Ile Ser Gly Leu Val Thr Asp
     50                  55                  60
Gln Leu Ser Ser Ser Ala Gln His Thr Gln Lys Thr Leu Cys Ser Leu
 65                  70                  75                  80
Arg Met Ala Phe Val Thr Ser Ser Ala Ala Ala Ser Val Leu Thr Val

85                  90                  95
Met Leu Ile Ala Phe Asp Arg Tyr Leu Ala Ile Lys Gln Pro Leu Arg
            100                 105                 110
Tyr Phe Gln Ile Met Asn Gly Leu Val Ala Gly Gly Cys Ile Ala Gly
        115                 120                 125
Leu Trp Leu Ile Ser Tyr Leu Ile Gly Phe Leu Pro Leu Gly Val Ser
    130                 135                 140
Ile Phe Gln Gln Thr Thr Tyr His Gly Pro Cys Thr Phe Phe Ala Val
145                 150                 155                 160
Phe His Pro Arg Phe Val Leu Thr Leu Ser Cys Ala Gly Phe Phe Pro
                165                 170                 175
Ala Val Leu Leu Phe Val Phe Phe Tyr Cys Asp Met Leu Lys Ile Ala
            180                 185                 190
Ser Val His Ser Gln His Ile Arg Lys Met Glu His Ala Gly Ala Met
        195                 200                 205
Val Gly Ala Cys Arg Pro Pro Arg Pro Val Asn Asp Phe Lys Ala Val
    210                 215                 220
Arg Thr Val Ser Val Leu Ile Gly Ser Phe Thr Leu Ser Trp Ser Pro
225                 230                 235                 240
Phe Leu Ile Thr Ser Ile Val Gln Val Ala Cys His Lys Cys Cys Leu
                245                 250                 255
Tyr Gln Val Leu Glu Lys Tyr Leu Trp Leu Leu Gly Val Gly Asn Ser
            260                 265                 270
Leu Leu Asn Pro Leu Ile Tyr Ala Tyr Trp Gln Arg Glu Val Arg Gln
        275                 280                 285
Gln Leu Cys His Met Ala Leu Gly Val Lys Lys Phe Phe Thr Ser Ile
    290                 295                 300
Phe Leu Leu Leu Ser Ala Arg Asn Arg Gly Pro Gln Arg Thr Arg Glu
305                 310                 315                 320
Ser Ser Tyr His Ile Val Thr Ile Ser Gln Pro Glu Leu Asp Gly
                325                 330                 335

<210>  5
<211>  28
<212>  DNA
<213>  Artificial Sequence

<220>
<223>  Description of Artificial Sequence: an artificially synthesized
       primer sequence

<400>  5
aaaagcttcc tgcagcctcc agctctcc                                        28

<210> 6
<211> 24
<212> DNA
<213> Artificial Sequence

<220>
<223> Description of Artificial Sequence: an artificially synthesized
      primer sequence

<400> 6
aaccatggcc tcttctgatg cagc                                              24


<210> 7
<211> 377
<212> DNA
<213> Homo sapiens

<400> 7
cctgcagcct ccagctctcc tggtctaatg tggaaagtgg cccaggtgag ggctttgctc      60

tcctggagac atttgccccc agctgtgagc agggacaggt ctggccaccg ggcccctggt     120

taagactcta atgacccgct ggtcctgagg aagaggtgct gacgaccaag gagatcttcc     180

cacagaccca gcaccaggga aatggtccgg aaattgcagc ctcagccccc agccatctgc     240

cgaccccccc accccaggcc ctaatgggcc aggcggcagg ggttgacagg taggggagat     300

gggctctgag actataaagc cagcgggggc ccagcagccc tcagccctcc aggacaggct     360

gcatcagaag aggccat                                                    377

## Description

### TECHNICAL FIELD

This invention relates to a screening tool, and a screening method, for an agent for increasing insulin content, on the basis of a promotion of insulin production, and a novel agent for increasing insulin content.

### BACKGROUND ART

Diabetes is defined as diseases which are characterized by chronic hyperglycemia caused by a deficiency of insulin action, and are accompanied by various characteristic metabolic disorders (non-patent reference 1). Diabetes is classified into two types, an "insulin dependent diabetes mellitus (type I)" characterized by a deficiency of insulin caused by a destructive lesion of pancreatic β cells, and a "noninsulin dependent diabetes mellitus (type II)" with a decreased sensibility to insulin and a decreased secretion of insulin.

In type II, which accounts for approximately 90% of patients suffering from diabetes, it is considered that chronic hyperglycemia causes a decrease in the action of pancreatic β cells, i.e., a decreased secretion of insulin and a decreased content of insulin (non-patent reference 2). Sulfonylureas are clinically used as a therapeutic agent for a diabetic patient with a decreased secretion of insulin, as it is known that sulfonylureas promote an insulin secretion from the pancreas without promoting an insulin biosynthesis, i.e., without increasing an insulin content. Further, it is known that sulfonylureas cause a functional disorder in pancreatic β cells, particularly a deficiency of insulin (non-patent reference 3). Therefore, agents capable of alleviating a decreased pancreatic function due to chronic hyperglycemia or use of sulfonylureas, particularly agents for promoting insulin production, increasing insulin content, and preventing and/or treating diabetes, are desired.

To increase insulin content in pancreatic β cells, it is necessary to promote an insulin biosynthesis by enhancing the transcriptional and/or translational processes of the insulin gene. Glucose and cAMP are known as compounds capable of promoting an insulin biosynthesis, and it is known that the action thereof is achieved by an increase in an amount of insulin mRNA caused by promoting transcription and stabilizing of the mRNA (non-patent references 4-6). Therefore, it is considered that substances capable of increasing insulin mRNA, such as substances capable of enhancing an insulin promoter activity, have an activity capable of increasing insulin content.

There is a known G protein-coupled receptor reported as a molecule involved in controlling an insulin secretion, i.e., a molecule having an activity of promoting an insulin secretion [International Publication WO02/44362 (patent reference 1)]. However, "an activity of promoting insulin production" and "an activity of increasing insulin content" are not known. Further, no assay appropriate to a screening of substances capable of increasing insulin content has been reported.

International Publication WO00/50562 (patent reference 2) discloses DNAs encoding polypeptides having sequences identical to those of human and rat receptors disclosed in International Publication WO02/44362, and polypeptides encoded by the DNAs. The reference further discloses that agonists and antagonists against the polypeptides can be applied to many of the same diseases, including diabetes.

European Patent Application Publication No. 1092727A (patent reference 3) and Japanese Unexamined Patent Publication (Kokai) 2001-186888 (patent reference 4) disclose a base sequence encoding an identical amino acid sequence as that of the above-mentioned human receptor, and an amino acid sequence in which an amino acid is deleted in the sequence disclosed in International Publication WO02/44362. The references further disclose that substances capable of controlling the polypeptides can be applied to the treatment of many diseases, and that it is preferable when treating diabetes.

International Publication WO01/32864 (patent reference 5), International Publication WO01/36473 (patent reference 6), International Publication WO01/42288 (patent reference 7), International Publication WO01/87929 (patent reference 8), International Publication WO02/64789 (patent reference 9), and International Publication WO02/61087 (patent reference 10) disclose the identical sequence of the above-mentioned human receptor, and further, disclose that substances capable of controlling the polypeptide can be applied to the treatment of many diseases including diabetes. Further, International Publication WO00/22131 (patent reference 11), International Publication WO00/31258 (patent reference 12), and International Publication WO02/16548 (patent reference 13) disclose the identical sequence of the above-mentioned human receptor, and further disclose that substances capable of controlling the polypeptide can be applied to the treatment of many diseases.

However, no references disclose or suggest that insulin production is promoted by activating the receptors, and that the receptors can be used as a tool to screen agents for promoting insulin production or agents for increasing insulin content, and further, neither disclose nor suggest a method that uses the receptors to screen agents for promoting insulin production or agents for increasing insulin content.
(non-patent reference 1) J. Japan Diab. Soc., 1999, 42(5), p.385-404
(non-patent reference 2) C.Ronald Kahn and Gordon C.Weir, ed, Yasunori Kanazawa and three colleagues, trans-ed., "JOSLIN'S DIABETES MELLITUS", Igaku-Shoin MYW, 1995, p.245-268
(non-patent reference 3) C.Ronald Kahn and Gordon C.Weir, ed, Yasunori Kanazawa and three colleagues, trans-ed., "JOSLIN'S DIABETES MELLITUS", Igaku-Shoin MYW, 1995, p.505-525
(non-patent reference 4) The Journal of Biological Chemistry, USA, 1985, 260, p.13585-13589
(non-patent reference 5) The Journal of Biological Chemistry, USA, 1985, 260, p.13590-13594
(non-patent reference 6) Diabetes, USA, 1977, 26, p.538-545
(patent reference 1) International Publication WO02/44362
(patent reference 2) International Publication WO00/50562
(patent reference 3) European Patent Application Publication No. 1092727A
(patent reference 4) Japanese Unexamined Patent Publication (Kokai) 2001-186888
(patent reference 5) International Publication WO01/32864
(patent reference 6) International Publication WO01/36473
(patent reference 7) International Publication WO01/42288
(patent reference 8) International Publication WO01/87929
(patent reference 9) International Publication WO02/64789
(patent reference 10) International Publication WO02/61087
(patent reference 11) International Publication WO00/22131
(patent reference 12) International Publication WO00/31258
(patent reference 13) International Publication WO02/16548

### DISCLOSURE OF INVENTION

The object of the present invention is to provide a tool useful in screening a substance which increases insulin content by promoting insulin production and is useful in preventing and/or treating diabetes, a screening method, and a novel agent for promoting insulin production and/or increasing insulin content.

It can be judged whether or not a substance promotes insulin production, by an activation of the insulin promoter gene as an indicator. Substances activating the insulin promoter gene include, for example, a substance which activates the insulin promoter gene through a factor activating the insulin promoter by direct binding thereto in a cell, or a substance which enhances the insulin promoter activity by directly affecting a protein such as a G protein-coupled receptor (GPCR) on the surface of a cell membrane, and thereby controlling the activity of the protein. Among substances enhancing the insulin promoter activity, a substance which functions in a cell must pass through a cell membrane (and further a nuclear membrane), while a substance which targets a protein on the surface of a cell membrane does not need to pass through a cell membrane. Since more than half of known medicaments target proteins on the surface of a cell membrane, such proteins are attractive as a target of medicaments, and are considered to be a target having a high potential for drug development. Accordingly, it is considered that a finding of a molecule (target molecule for drug development) which is a protein located in a cell membrane and capable of controlling the activity of the insulin promoter gene is very important in the developing of a therapeutic agent for diabetes on the basis of increasing insulin content, and can contribute to the prevention and treatment of diabetes.

The present inventors have conducted intensive studies and, as a result, found that the activation of GPCR consisting of the amino acid sequence of SEQ ID NO: 2 increases the insulin promoter activity, and thereby the promotion of insulin production is increased. Further, on the basis of the finding, the present inventors provided a screening method, using the GPCR, for an agent for promoting insulin production and/or an agent for increasing insulin content. Furthermore, the present inventors confirmed that substances activating the GPCR consisting of the amino acid sequence of SEQ ID NO: 2 actually increased the insulin promoter activity, and provided novel agents for promoting insulin production, and thus the present invention was completed.

The present invention relates to:
[1] a screening tool for an agent for promoting insulin production and/or an agent for increasing insulin content, wherein the tool is a polypeptide comprising (1) the amino acid sequence of SEQ ID NO: 2 or 4 or (2) an amino acid sequence in which 1 to 15 amino acids are deleted, substituted, and/or inserted in the amino acid sequence of SEQ ID NO: 2 or 4, and exhibiting an activity of promoting insulin production by activation;
[2] a screening tool for an agent for promoting insulin production and/or an agent for increasing insulin content, wherein the tool is a polypeptide comprising the amino acid sequence of SEQ ID NO: 2 or 4 and exhibiting an activity of promoting insulin production by activation;
[3] a screening tool for an agent for promoting insulin production and/or an agent for increasing insulin content, wherein the tool is a polypeptide consisting of an amino acid sequence having an 80% or more homology with that of SEQ ID NO: 2 or 4, and exhibiting an activity of promoting insulin production by activation;
[4] a screening tool for an agent for promoting insulin production and/or an agent for increasing insulin content, wherein the tool is a polypeptide consisting of the amino acid sequence of SEQ ID NO: 2 or 4 (hereinafter, the screening tools of [1] to [4] for an agent for promoting insulin production and/or an agent for increasing insulin content are collectively referred to as a polypeptide-type screening tool);
[5] a screening tool for an agent for promoting insulin production and/or an agent for increasing insulin content, wherein the tool is a cell expressing the polypeptide of [1] to [4] (hereinafter referred to as a cell-type screening tool);
[6] use of the polypeptide of [1] to [4] or the cell of [5] for screening an agent for promoting insulin production and/or an agent for increasing insulin content;
[7] a method for screening an agent for promoting insulin production and/or an agent for increasing insulin content, comprising the steps of:
   bringing the cell of [5] or a cell membrane thereof into contact with a substance to be tested, and
   analyzing whether or not the polypeptide of [1] to [4] is activated;
[8] a process for manufacturing a pharmaceutical composition for promoting insulin production and/or increasing insulin content, comprising the steps of:
   bringing the cell of [5] or a cell membrane thereof into contact with a substance to be tested,
   analyzing whether or not the polypeptide of [1] to [4] is activated, and
   preparing a medicament containing the substance;
[9] an agent for promoting insulin production and/or an agent for increasing insulin content, comprising as an active ingredient a substance activating the polypeptide of [1] to [4];
[10] a method for promoting insulin production and/or increasing insulin content, comprising administering to a subject a substance activating the polypeptide of [1] to [4];
[11] use of a substance activating the polypeptide of [1] to [4], in the manufacture of a pharmaceutical composition for promoting insulin production and/or increasing insulin content.

The term "screening tool" as used herein means a tool used for screening, more particularly, a polypeptide or a cell expressing a polypeptide used for screening. The term "screening tool for an agent for promoting insulin production and/or an agent for increasing insulin content" as used herein means a polypeptide or a cell used as a subject to be brought into contact with a test substance, for screening an agent for promoting insulin production and/or an agent for increasing insulin content. The term "agent for promoting insulin production and/or agent for increasing insulin content" mean an agent for promoting insulin production, an agent for increasing insulin content, and an agent for increasing insulin content on the basis of promoting insulin production.

### BRIEF DESCRIPTION OF THE DRAWINGS

Figure 1 is a graph showing a luciferase activity in mouse pancreatic β cell strain NIT1 cells transfected with plasmid pEF-BOS SSF-NA or a control vector (plasmid pEF-BOS), together with plasmid InsPro. The vertical axis shows a luciferase activity.
Figure 2 is a graph showing the results of a luciferase activity measured after treating plasmid InsPro-transfected mouse pancreatic β cell strain NIT1 cells with 2-(pyridine-4-yl)ethyl thiobenzoate (hereinafter referred to as compound A). The vertical axis shows a luciferase activity and the horizontal axis shows a compound concentration (µmol/L).
Figure 3 is a graph showing the results of a luciferase activity measured after treating plasmid InsPro-transfected mouse pancreatic β cell strain NIT1 cells with 4-{5-[(E)-(1,3-diethyl-5-oxo-2-thioxoimidazolidine-4-ylidene)methyl]-2-furyl}benzoic acid (hereinafter referred to as compound B),
   (2Z)-2,3-bis(3,4-dimethoxyphenyl)acrylonitrile (hereinafter referred to as compound C),
   4-[(E)-2-(3,4-dimethoxyphenyl)vinyl]pyridine (hereinafter referred to as compound D), or
   5-{[4-(3-methyl-1,2,4-oxadiazole-5-yl)benzyl]thio}-1H-1,2,4-triazole-3-amine (hereinafter referred to as compound E). The vertical axis shows a luciferase activity, and the symbol "R" means a control.

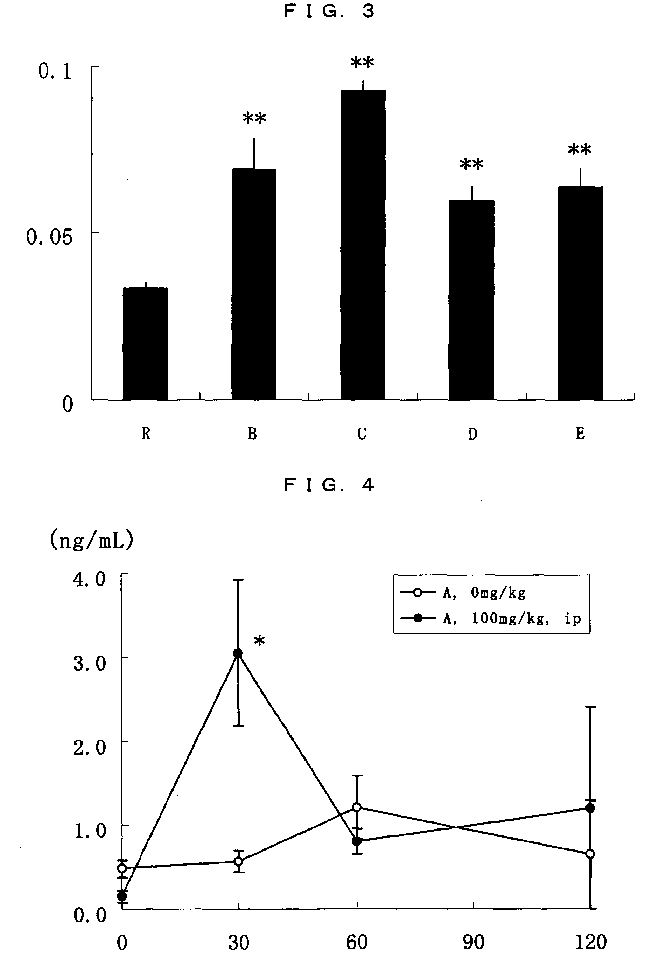

Figure 4 is a graph showing a time course of the concentration of plasma insulin after the oral administration of glucose in SD rats to which compound A was intraperitoneally administered or not administered. The vertical axis shows insulin in plasma (ng/mL), and the horizontal axis shows time after the oral administration of glucose (min.).

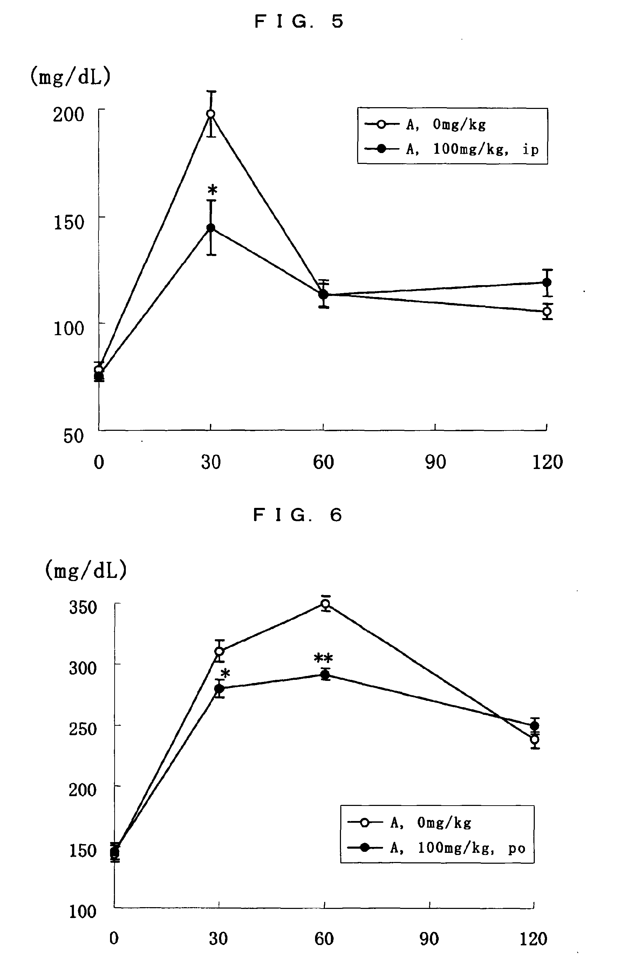

Figure 5 is a graph showing a time course of the blood glucose level after the oral administration of glucose in SD rats to which compound A was intraperitoneally administered or not administered. The vertical axis shows the glucose level (mg/dL), and the horizontal axis shows time after the oral administration of glucose (min.).
Figure 6 is a graph showing a time course of a blood glucose level after the oral administration of glucose in GK rats to which compound A was orally administered or not administered. The vertical axis shows the glucose level (mg/dL), and the horizontal axis shows time after the oral administration of glucose (min.).

### BEST MODE FOR CARRYING OUT THE INVENTION

### 1. Screening tool of the present invention

The screening tool of the present invention for an agent for promoting insulin production and/or an agent for increasing insulin content includes a polypeptide-type screening tool and a cell-type screening tool.

### (1) Polypeptide-type screening tool

As the polypeptide which may be used as the polypeptide-type screening tool of the present invention, there may be mentioned, for example,
(i) a polypeptide consisting of the amino acid sequence of SEQ ID NO: 2 or 4;
(ii) a polypeptide comprising the amino acid sequence of SEQ ID NO: 2 or 4, or an amino acid sequence in which 1 to 15 amino acids are deleted, substituted, and/or inserted in the amino acid sequence of SEQ ID NO: 2 or 4, and exhibiting an activity of promoting insulin production by activation (hereinafter referred to as a variation functionally equivalent); and
(iii) a polypeptide consisting of an amino acid sequence having an 80% or more homology with that of SEQ ID NO: 2 or 4, and exhibiting an activity of promoting insulin production by activation (hereinafter referred to as a homologous polypeptide). Hereinafter, the polypeptides which may be used as the polypeptide-type screening tool of the present invention are collectively referred to as polypeptides for a screening tool.

The polypeptide consisting of the amino acid sequence of SEQ ID NO: 2, which is a polypeptide for a screening tool, is a human G protein-coupled receptor consisting of 335 amino acid residues. Further, the polypeptide consisting of the amino acid sequence of SEQ ID NO: 4, which is a polypeptide for a screening tool, is a rat G protein-coupled receptor consisting of 335 amino acid residues. The homology between the human polypeptide consisting of the amino acid sequence of SEQ ID NO: 2 and the rat polypeptide consisting of the amino acid sequence of SEQ ID NO: 4 is 80.6% in the amino acid sequence comparison.

The term "homology" as used herein means a value obtained by a BLAST [Basic local alignment search tool; Altschul, S. F. et al., J. Mol. Biol., 215, 403-410, (1990)]. The homology in the amino acid sequence may be calculated by a BLAST search algorithm. More particularly, it may be calculated by using a bl2seq program (Tatiana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. The value means "identity" obtained when a program "blastp" as a pairwise alignment parameter, "0" as a Gap insertion cost value, "0" as a Gap elongation cost value, "SEG" as a filter for a Query sequence, and "BLOSUM62" as a Matrix are used, respectively.

The polypeptides consisting of the amino acid sequence of SEQ ID NO: 2 or 4 exhibit an activity of promoting insulin production by activation (hereinafter sometimes simply referred to as "the activity of promoting insulin production").

A method for confirming whether or not a polypeptide exhibits "an activity of promoting insulin production by activation" as used herein is not particularly limited, but it may be confirmed by, for example, the method described below, preferably a method described in Example 3. Namely, cells are respectively transformed with an expression vector comprising a polynucleotide encoding the polypeptide or a control expression vector without the polynucleotide, together with a plasmid in which a reporter gene (such as a luciferase gene) is linked downstream of an insulin promoter. After a predetermined number of hours (such as 24 hours) from the transformation, the medium is removed, the cells are lysed with a cell lysing solution, and a reporter activity of each lysate is measured. When the reporter activity in the lysate of the cells transformed with the expression vector comprising the polynucleotide encoding the polypeptide (test cells) is increased, in comparison with that of the cells transformed with the control expression vector (control cells), it may be judged that the polypeptide exhibits the "activity of promoting insulin production by activation".

The state in which the polypeptide for a screening tool, a G protein-coupled receptor, is "activated" as used herein means a state in which a signal is transduced downstream of the G protein-coupled receptor regardless of a ligand binding. The polypeptide is activated when the total amount of an active form of G protein-coupled receptor exceeds a certain amount.

G protein-coupled receptors are in state of equilibrium between an active form and an inactive form.
The equilibrium shifts to the active form when a ligand binds to the G protein-coupled receptor. It is known that the G protein-coupled receptor is also activated and transduces a signal downstream thereof in the absence of the ligand when the G protein-coupled receptor is overexpressed, because the total amount of the activated G protein-coupled receptor increases (Milano, C. A. et al., Science, 264, 582-586, 1994). Therefore, even if the ligand is not identified, it is possible to detect a signal from the G protein-coupled receptor by overexpressing the G protein-coupled receptor in cells. In the experiment described in Example 3, the polypeptide for a screening tool is activated in the absence of the ligand thereagainst by an overexpression of polypeptide. The state is the same as that activated by an agonist binding.

The variation functionally equivalent which may be used as the polypeptide-type screening tool of the present invention may be a naturally-occurring sequence or an artificially synthesized sequence, and an origin thereof is not particularly limited.

The variation functionally equivalent includes, not only human variations of the polypeptide consisting of the amino acid sequence of SEQ ID NO: 2 or rat variations of the polypeptide consisting of the amino acid sequence of SEQ ID NO: 4, but also variations functionally equivalent derived from organisms other than a human or a rat (such as a mouse, a hamster, or a dog), and further, polypeptides obtained by artificially modifying these native polypeptides (i.e., human or rat variations, or variations functionally equivalent derived from organisms other than a human or a rat) or the polypeptide consisting of the amino acid sequence of SEQ ID NO: 2 or 16 by genetic engineering techniques, so long as the above definition of the variation functionally equivalent is met. The term "variation" as used herein means an individual difference between the same polypeptides in the same species or a difference between homologous polypeptides in several species.

As the variation functionally equivalent, a polypeptide consisting of an amino acid sequence in which 1 to 15 in total, preferably 1 to 10, more preferably 1 to 7, most preferably 1 to 5 amino acids are deleted, substituted, inserted, and/or added at one or plural positions in the amino acid sequence of SEQ ID NO: 2 or 4, or comprising the amino acid sequence of SEQ ID NO: 2 or 4, and exhibiting the activity of promoting insulin production is preferable.

The polypeptide comprising the amino acid sequence of SEQ ID NO: 2 or 4 and exhibiting the activity of promoting insulin production includes, for example, a polypeptide in which an appropriate marker sequence or the like is added to the N-terminus and/or the C-terminus of the polypeptide consisting of the amino acid sequence of SEQ ID NO: 2 or 4 (i.e., fusion polypeptide), so long as it exhibits the activity of promoting insulin production.

As the marker sequence, a sequence for easily carrying out a confirmation of polypeptide expression, a confirmation of intracellular localization thereof, a purification thereof, or the like may be used. As the sequence, there may be mentioned, for example, a FLAG epitope, a hexa-histidine tag, a hemagglutinin tag, a myc epitope, or the like.

The homologous polypeptide which may be used as the polypeptide-type screening tool of the present invention is not particularly limited, so long as it is a polypeptide consisting of an amino acid sequence having an 80% or more homology with the amino acid sequence of SEQ ID NO: 2 or 4, and exhibiting the activity of promoting insulin production. The homologous polypeptide may consists of an amino acid sequence having preferably a 90% or more homology, more preferably a 95% or more homology, still further preferably a 98% or more homology, most preferably a 99% or more homology, with respect to the amino acid sequence of SEQ ID NO: 2 or 4.

The polypeptide for a screening tool which may be used as the polypeptide-type screening tool of the present invention may be obtained by various known methods, such as methods described in International Publication WO02/44362.

### (2) Cell-type screening tool

The cell which may be used as the cell-type screening tool of the present invention (hereinafter referred to as a cell for a screening tool) is not particularly limited, so long as it expresses the polypeptide for a screening tool when using as the cell-type screening tool. The cell for a screening tool may be a cell transformed with the above-mentioned expression vector, or a naturally occurring cell known to express the polypeptide for a screening tool, or a cell line thereof (such as pancreatic β cell line, preferably an NIT1 cell).

As the cell for a screening tool which may be used as the cell-type screening tool of the present invention, transformants are preferable, such as
(i) a transformant expressing the polypeptide consisting of the amino acid sequence of SEQ ID NO: 2 or 4;
(ii) a transformant expressing the variation functionally equivalent; and
(iii) a transformant expressing the homologous polypeptide.

The cell for a screening tool may be obtained, for example, by re-integrating a polynucleotide encoding the polypeptide for a screening tool into an appropriate vector DNA, and transforming a host cell (preferably eukaryotic host cell, most preferably 293-EBNA cell). Further, it is possible to express the polynucleotide in a desired host cell, by introducing an appropriate promoter and a sequence related to the gene expression into the vector.

The cell transformed with the expression vector may be, for example, a cell in which a polynucleotide encoding the polypeptide for a screening tool is integrated into a chromosome of a host cell, or a cell containing the polynucleotide as an expression vector comprising the polynucleotide. The cell for a screening tool may be obtained, for example, by transforming a desired host cell with an expression vector comprising a polynucleotide encoding the polypeptide for a screening tool. More particularly, it may be prepared in accordance with methods described in International Publication WO02/44362.

### 2. Screening method for agent for promoting insulin production and/or agent for increasing insulin content

It is possible to screen a substance capable of controlling activities of the polypeptide for a screening tool (particularly a substance activating the polypeptide for a screening tool, i.e., agonist), using the polypeptide for a screening tool or the cell for a screening tool. As described above, the polypeptide for a screening tool has an activity of promoting insulin production by activation. Therefore, a substance activating the polypeptide for a screening tool is useful as an active ingredient of an agent for increasing insulin content, capable of promoting insulin production. Further, the polypeptide for a screening tool per se or the cell for a screening tool per se may be used as a tool for screening an agent for promoting insulin production and/or an agent for increasing insulin content.

The term "promoting insulin production" as used herein means that a condition in which the activity of promoting insulin production is significantly increased with respect to a control group, and the activity of promoting insulin production in a group treated with a test substance with respect to that in the control group is 1.5 times or more (preferably 5 times or more).

Substances to be tested which may be screened by using the screening tool of the present invention are not particularly limited, but there may be mentioned, for example, various known compounds (including peptides) registered in chemical files, compounds obtained by combinatorial chemistry techniques (Terrett, N. K. et al., Tetrahedron, 51, 8135-8137, 1995), random peptides prepared by employing a phage display method (Felici, F. et al., J. Mol. Biol., 222, 301-310, 1991) or the like, culture supernatants of microorganisms, natural components derived from plants or marine organisms, animal tissue extracts, or the like. Further, compounds (including peptides) obtained by chemically or biologically modifying compounds (including peptides) selected by the screening tool of the present invention for an agent for promoting insulin production and/or an agent for increasing insulin content may be used.

The screening method of the present invention is not particularly limited, so long as it comprises the steps of bringing the cell for a screening tool in which the polypeptide for a screening tool is expressed and functions as a receptor, or a cell membrane thereof, into contact with a substance to be tested and analyzing whether or not the polypeptide is activated. There may be mentioned, on the basis of differences in methods used for analyzing an activation of the polypeptide, for example,
1) a screening method in which changes of an intracellular cAMP concentration are used as an indicator (hereinafter referred to as "cAMP-type screening method"),
2) a screening method using a GTPγS binding method (hereinafter referred to as "GTPγS binding-type screening method"),
3) a screening method using a ligand binding assay method (hereinafter referred to as "ligand binding-type screening method"), or
4) a method in which an insulin promoter activity is used as an indicator (hereinafter referred to as "insulin promoter activity-type screening method").

In the screening method of the present invention, it is preferable to use the cAMP-type screening method (particularly the method described in Example 4) or the insulin promoter activity-type screening method (particularly the method described in Example 5), and more preferable to use the combination of the cAMP-type screening method (particularly the method described in Example 4) and the insulin promoter activity-type screening method (particularly the method described in Example 5).

Further, when a naturally-occurring cell or a cell line thereof (i.e., not transformant) is used in the screening, it is preferable to confirm that the substance obtained by the screening can activate the polypeptide for a screening tool, by using the above-mentioned transformant (i) to (iii) as a screening tool.

### 1) cAMP-type screening method

In the case of screening a substance activating the polypeptide for a screening tool (i.e., agonist) which is useful as an active ingredient of an agent for promoting insulin production and/or an agent for increasing insulin content by the use of changes of an intracellular cAMP concentration as an indicator, it is analyzed whether or not the polypeptide is activated by bringing the cell for a screening tool into contact with a test substance, and analyzing (i.e., measuring or detecting) changes of the intracellular cAMP concentration in the cells, directly or indirectly. Namely, the cAMP-type screening method of the present invention in which changes of the intracellular cAMP concentration are used as an indicator comprises the steps of bringing the cell for a screening tool into contact with a test substance, and analyzing changes of the intracellular cAMP concentration in the cells. More particularly, the screening is preferably carried out by the method described in Example 4. For example, an increase in the intracellular cAMP concentration, as an indicator, is measured by exposing a test substance for a predetermined time, and then a test substance in which an increased activity in the cell for a screening tool is 1.5 times or more (preferably 5 times or more) with respect to that in the control cell may be selected as a substance having an agonist activity.

Changes of the intracellular cAMP concentration may be, for example, directly analyzed by the use of a commercially available a cAMP measuring kit (Amersham or the like), or indirectly analyzed by analyzing a transcriptional activity of a gene in which a regulation of the transcription is dependent on the cAMP concentration [such as a gene obtained by introducing a cAMP responsive element (CRE) upstream of a luciferase gene] as shown in Example 4.

When the cell for a screening tool is brought into contact with a test substance, and the intracellular cAMP concentration therein is increased, it may be judged that the test substance is an agonist against the polypeptide for a screening tool. In this connection, a similar procedure is carried out using, as a control, a host cell not expressing the polypeptide for a screening tool or a cell transformed with empty vector instead of the cell for a screening tool, and it is preferable to confirm that the cAMP concentration in the control cells is not increased by the test substance.

The screening for a substance activating the polypeptide for a screening tool by directly analyzing changes of the cAMP concentration using a commercially available cAMP measuring kit (Amersham or the like) may be carried out by, for example, the following procedure. More particularly, cells containing a gene encoding the polypeptide for a screening tool are cultured for 20 hours after the gene transfer, and the medium is removed. After 400 µL of 1 mmol/L IBMX (3-isobutyl-1-methylxanthine)/DMEM is added, the whole is incubated at 37°C for 10 minutes in the presence of 5% CO₂. Further, a test substance (such as a compound, a peptide, an antibody, or the like) diluted with 100 µL of 1 mmol/L IBMX/DMEM is added and incubated for 30 minutes. The medium is removed, and then an amount of cAMP in the resulting cells is measured using a commercially available cAMP measuring kit (such as cAMP enzymeimmunoassay system; Amersham pharmacia biotech). A test substance in which a specific increase of the cAMP in the presence of the test substance is observed may be screened as a substance activating the polypeptide for a screening tool, i.e., an agent for promoting insulin production and/or an agent for increasing insulin content.

The screening for a substance activating the polypeptide for a screening tool by indirectly analyzing changes of the cAMP concentration by analyzing a transcriptional activity of a gene in which a regulation of the transcription is dependent on the cAMP concentration may be carried out by, for example, the following procedure as shown in Example 4. More particularly, cells containing a gene encoding the polypeptide for a screening tool and a gene in which a regulation of the transcription is dependent on the cAMP concentration [for example, a gene obtained by introducing a cAMP responsive element (CRE) upstream of a luciferase gene; such as a pCRE-Luc vector (CLONTECH)] are cultured for 18 to 20 hours after the gene transfer. A test substance diluted with a medium is added and the whole is incubated at 37°C for 5 to 6 hours in the presence of 5% CO₂. The medium is removed, and the cells are lysed with a cell lysing solution. A luciferase activity of the lysate is measured. A substance or the like in which a specific increase of a reporter activity in the presence of the test substance is observed may be screened as a substance activating the polypeptide for a screening tool, i.e., an agent for promoting insulin production and/or an agent for increasing insulin content.

### 2) GTPγS binding-type screening method

The screening for a substance activating the polypeptide for a screening tool (i.e., agonist) which is useful as an active ingredient of an agent for promoting insulin production and/or an agent for increasing insulin content using a GTPγS binding method (Lazareno, S. and Birdsall, N. J. M., Br. J. Pharmacol., 109, 1120-1127, 1993) may be carried out by, for example, the following procedure. More particularly, a cell membrane expressing the polypeptide for a screening tool is mixed with ³⁵S labeled GTPγS (400 pmol/L) in a mixing solution [20 mmol/L HEPES (pH 7.4), 100 mmol/L NaCl, 10mmol/L MgCl₂, and 50 mmol/L GDP]. After incubation in the presence or absence of a test substance, reaction solutions are filtered with a glass filter or the like, and then the remaining GTPγS radioactivity on each filter is measured by a liquid scintillation counter or the like. An agonist against the polypeptide for a screening tool, i.e., an agent for promoting insulin production and/or an agent for increasing insulin content, may be screened by a specific increase of the GTPγS binding in the presence of a test substance as an indicator.

The GTPγS binding-type screening method of the present invention using the GTPγS binding method comprises the steps of bringing a cell membrane of the cell for a screening tool into contact with a test compound in the presence of ³⁵S labeled GTPγS, separating the GTPγS binding to the cell membrane from the unbound GTPγS, and analyzing a radioactivity of one of the separated GTPγSs.

### 3) Ligand binding-type screening method

The screening for a substance binding to the polypeptide for a screening tool which is useful as an active ingredient of an agent for promoting insulin production and/or an agent for increasing insulin content using a ligand binding assay method may be carried out by, for example, the following procedure. More particularly, the cell for a screening tool expressing the polypeptide for a screening tool, or a cell membrane thereof, or the polypeptide for a screening tool (preferably a purified preparation thereof) is prepared. Assay conditions such as a buffer, ions, and/or pH are optimized. The transformant expressing the polypeptide, or the cell membrane thereof, or the polypeptide, and a labeled substance obtained by, for example, the cAMP-type screening method and/or the GTPγS binding-type screening method (i.e., an agonist) are incubated in the optimized buffer, together with a test substance, for a predetermined time. After the reaction, the whole is filtered with a glass filter or the like, and the filter is washed with an appropriate volume of the buffer. The remaining radioactivity on the filter is measured by a liquid scintillation counter or the like. A ligand of the polypeptide for a screening tool may be selected by the binding inhibition of the label as an indicator. In this connection, it may be confirmed that the ligand is an agonist or an antagonist by, for example, the cAMP-type screening method and/or the GTPγS binding-type screening method.

### 4) Insulin promoter activity-type screening method

In the case of screening a substance activating the polypeptide for a screening tool (i.e., agonist) which is useful as an active ingredient of an agent for promoting insulin production and/or an agent for increasing insulin content, on the basis of an insulin promoter activity as an indicator, it is analyzed whether or not the polypeptide is activated by bringing the cell for a screening tool into contact with a test substance, and analyzing (i.e., measuring or detecting) changes of the insulin promoter activity in the cells.

Changes of the insulin promoter activity may be analyzed, for example, by analyzing the transcriptional activity of a reporter gene (such as a luciferase gene), using a plasmid in which the reporter gene is linked downstream of the insulin promoter, as shown in Example 5.

More particularly, for example, a plasmid in which a reporter gene (such as a luciferase gene) is linked downstream of the insulin promoter is introduced into the cell for a screening tool, and the resulting cells are cultured for 18 to 20 hours. After a test substance diluted with the medium is added, the cells are further incubated at 37°C for 24 hours in the presence of 5% CO₂. The medium is aspirated, and the cells are lysed with a cell lysing solution. The reporter activity (such as a luciferase activity) of the lysate is measured. A substance or the like in which a specific increase of the reporter activity in the presence of the test substance is observed may be screened as a substance activating the polypeptide for a screening tool, i.e., an agent for promoting insulin production and/or an agent for increasing insulin content. In this connection, the similar procedure is carried out using, as a control, a cell not expressing the polypeptide for a screening tool instead of the cell for a screening tool, and it is preferable to confirm that the insulin promoter reporter activity in the control cells is not increased by the test substance.

### 3. Pharmaceutical compound for promoting insulin production and/or for increasing insulin content

The present invention includes a pharmaceutical composition for promoting insulin production and/or for increasing insulin content comprising as an active ingredient a substance [for example, DNAs, proteins (including antibodies and fragments thereof), peptides, or other compounds] activating the polypeptide for a screening tool, for example, selected by the screening method of the present invention. As the active ingredient, there may be mentioned, for example, 2-(pyridine-4-yl)ethyl thiobenzoate, 4-{5-[(E)-(1,3-diethyl-5-oxo-2-thioxoimidazolidine-4-ylidene)methyl]-2-furyl}benzoic acid, (2Z)-2,3-bis(3,4-dimethoxyphenyl)acrylonitrile, 4-[(E)-2-(3,4-dimethoxyphenyl)vinyl]pyridine, or 5-{[4-(3-methyl-1,2,4-oxadiazole-5-yl)benzyl]thio}-1H-1,2,4-triazole-3-amine, described in Example 6, or the like.

Further, the present invention includes a process for manufacturing a pharmaceutical composition for promoting insulin production and/or for increasing insulin content consisting of the steps of:
performing an analysis as described below in a quality control test of a pharmaceutical composition for promoting insulin production and/or for increasing insulin content; and
preparing a medicament containing the analyzed substance. The analysis may be carried out by
   (1) bringing a cell for a screening tool or a cell membrane thereof into contact with a test substance, and analyzing whether or not a polypeptide for a screening tool is activated; or
   (2) bringing a cell for a screening tool or a cell membrane thereof into contact with a test substance in the presence of a labeled agonist of a polypeptide for a screening tool, and analyzing a change of an amount of the labeled agonist which binds to the cell or the cell membrane thereof.

Further, the present invention includes a process for manufacturing a pharmaceutical composition for promoting insulin production and/or for increasing insulin content consisting of the step of preparing a medicament containing a substance obtained by the screening method of the present invention comprising the analysis by the above-mentioned procedures.

As an active ingredient in the pharmaceutical composition of the present invention, a substance activating the polypeptide for a screening tool may be used. The activating substance may be selected by, for example, the screening method of the present invention. The pharmaceutical composition of the present invention is not limited to a pharmaceutical composition comprising as an active ingredient a substance obtained by the screening method of the present invention, but includes a pharmaceutical composition for promoting insulin production and/or for increasing insulin content comprising as an active ingredient a substance activating the polypeptide for a screening tool.

In this connection, it is possible to confirm that the insulin production and the insulin content are increased by methods known to those skilled in the art, or by modified methods. For example, it may be confirmed by repetitively administrating a substance activating the polypeptide for a screening tool to a diabetes model animal, and measuring an amount of the insulin mRNA or insulin protein in the pancreas. Further, under the same conditions, effects of the substance on the treatment of diabetes may be judged by confirming the hypoglycemic effects in a non-fasting diabetes model animal in accordance with a conventional method, or by confirming a function to suppress the increased blood glucose level after a glucose tolerance test by an oral dose.

The preparation comprising as an active ingredient a substance [for example, DNAs, proteins (including antibodies and fragments thereof), peptides, or other compounds] activating the polypeptide for a screening tool may be prepared, as a pharmaceutical composition, using pharmaceutically acceptable carriers, fillers, and/or other additives generally used in the preparation of formulation, in accordance with the active ingredient. The present invention includes a method for promoting insulin production and/or increasing insulin content, comprising administering to a subject in need thereof a substance activating the polypeptide for a screening tool in an amount effective therefor. Further, the present invention includes the use of a substance activating the polypeptide for a screening tool, in the manufacture of a pharmaceutical composition for promoting insulin production and/or increasing insulin content.

Examples of administration include oral administration by tablets, pills, capsules, granules, fine granules, powders, oral solutions and the like, and parenteral administration by injections (e.g., intravenous, intramuscular, or the like), suppositories, transdermal preparations, transmucosal absorption preparations and the like. Particularly, in the case of peptides which are digested in the stomach, a parenteral administration such as an intravenous injection or the like is preferable.

In the solid composition for use in the oral administration, one or more active substances may be mixed with at least one inert diluent such as lactose, mannitol, glucose, microcrystalline cellulose, hydroxypropylcellulose, starch, polyvinyl pyrrolidone, or aluminum magnesium silicate. In the usual way, the composition may contain additives other than the inert diluent, such as a lubricant, a disintegrating agent, a stabilizing agent, or a solubilizing or solubilization assisting agent. If necessary, tablets or pills may be coated with a sugar coating or a film of a gastric or enteric substance.

The liquid composition for oral administration may include, for example, emulsions, solutions, suspensions, syrups, and elixirs, and may contain a generally used inert diluent such as purified water or ethyl alcohol. The composition may contain additives other than the inert diluent, such as moistening agents, suspending agents, sweeteners, flavors, or antiseptics.

The injections for parenteral administration may include aseptic aqueous or non-aqueous solutions, suspensions, and emulsions. Examples of the diluent for use in the aqueous solutions and suspensions include distilled water for injection use and physiological saline. Examples of the diluent for use in the non-aqueous solutions and suspensions include propylene glycol, polyethylene glycol, plant oil (e.g., olive oil), alcohols (e.g., ethanol), polysorbate 80 and the like. Such a composition may further contain a moistening agent, an emulsifying agent, a dispersing agent, a stabilizing agent, a solubilizing or solubilization assisting agent, an antiseptic or the like. These compositions may be sterilized, for example, by filtration through a bacteria retaining filter, blending of a germicide, or irradiation. Alternatively, they may be used by first making into sterile solid compositions and dissolving them in sterile water or other sterile solvent for injection use prior to their use.

The dose is optionally decided by taking into consideration the strength of each active ingredient, or the symptoms, age, sex, or the like of each patient to be administered.

For example, in the case of oral administration, the usual dosage for an adult (60 kg in weight) is about 0.1 to 100 mg, preferably 0.1 to 50 mg per day. In the case of parenteral administration, the usual dosage is about 0.01 to 50 mg, preferably 0.01 to 10 mg per day in the form of an injection.

### EXAMPLES

The present invention now will be further illustrated by, but is by no means limited to, the following Examples. The procedures were performed in accordance with the known methods (for example, Maniatis, T., et al., "Molecular Cloning - A Laboratory Manual", Cold Spring Harbor Laboratory, NY, 1982; and Hille, B., Ionic Channels of Excitable Membranes, 2nd Ed., Sinauer Associates Inc., MA, 1992), unless otherwise specified.

### Example 1: Preparation of expression vector comprising polypeptide consisting of amino acid sequence of SEQ ID NO: 2

In accordance with the procedure described in Example 1 of International Publication WO02/44362, a DNA having the base sequence of SEQ ID NO: 1 was obtained, and was introduced into plasmid pEF-BOS (hereinafter referred to as plasmid pEF-BOS-NA). Then, to express the polypeptide consisting of the amino acid sequence of SEQ ID NO: 2, a pEF-BOS signal sequence flag plasmid into which the full-length DNA encoding the polypeptide consisting of the amino acid sequence of SEQ ID NO: 2 was prepared (hereinafter referred to as plasmid pEF-BOS SSF-NA). This was because the expression vector capable of adding a signal sequence to the N-terminus of the desired polypeptide was used to express the desired polypeptide at a high frequency on a cell membrane.

### Example 2: Construction of human insulin promoter reporter plasmid

The base sequence of the 5' expression regulatory region of human insulin gene was identified (Nature, 284, 26-32, 1980), and plural cis elements known as a transcription factor binding site, commonly exist in the 5' expression regulatory region of mouse or rat insulin gene, as well as that of the human insulin gene (Diabetes, 44, 1002-1004, 1995). A polymerase chain reaction (PCR) was performed using a region comprising the cis elements common to these species and considered enough to exhibit a promoter activity, so that the HindIII site and the NcoI site were generated at the 5' and 3' sides thereof, respectively, and the amplified fragment was cloned to plasmid pCR2.1-Topo (Cat. No. K455001, TA cloning system; Invitrogen). As the region, the region between -342 and +37 was used in the present example ["+1" denotes the putative transcription initiation point shown in Proc. Natl. Acad. Sci. U.S.A., 95, 11572-11577, 1998].

The PCR was carried out under the following conditions. A cycle consisting of the steps of denaturing double stranded DNAs at 94°C for 30 seconds, annealing primers with denatured single DNAs at 55°C for 30 seconds, and elongating DNAs at 72°C for 1 minute, was repeated 30 times, using DNA polymerase (Ampli Taq DNA polymerase; Applied Biosystems). The base sequences of primers [Ins17(h) and Ins19(h)] used in the PCR are shown in SEQ ID NOS: 5 and 6.

The cloned plasmid with the amplified fragment was digested with restriction enzymes HindIII(Takara Shuzo) and NcoI (Takara Shuzo) to excise the amplified fragment from the plasmid. The excised fragment was cloned into a plasmid containing the luciferase gene (Cat. No. 306-04831, luciferase vector pGV-B2; Toyo Ink MFG), between the NcoI cite in the initiation codon of the luciferase gene and the HindIII site located at the 5' upstream thereof. The base sequence of the cloned human insulin promoter was determined using a DNA sequencer (ABI377 DNA Sequencer; Applied Biosystems) by a dideoxy terminator method. The determined base sequence is shown in SEQ ID NO: 7.

As described above, the human insulin promoter reporter plasmid pIns-Luc380 (hereinafter referred to plasmid InsPro) was constructed. When the plasmid is introduced into a cell and the insulin promoter region contained in the plasmid is activated, the luciferase gene is biosynthesized. The insulin promoter activity can be measured by measuring the luciferase activity.

### Example 3: Change of insulin promoter reporter activity by overexpression of polypeptide consisting of amino acid sequence of SEQ ID NO: 2

It is known that cAMP is a second messenger of the polypeptide consisting of the amino acid sequence of SEQ ID NO: 2 (see Example 4 in International Publication WO02/44362). In the present example, effects of overexpression of the polypeptide on an insulin promoter activity were examined.

Mouse pancreatic β cell line NIT1 cells (4×10⁴ cells/well; ATCC: CRL-2055) were seeded on 96-well plate, and cultured in an F-12 medium containing 10% fetal calf serum (FCS) overnight. Then, a transfection reagent (FuGENE6; Boeringer Mannheim) was used to transfect the cells with plasmid InsPro (1 ng) prepared in Example 2 and plasmid pEF-BOS SSF-NA (10 ng). In this connection, as a control, transfection with plasmid InsPro and plasmid pEF-BOS (control vector) was carried out. After the transfection, the cells were cultured for 24 hours, and the medium was aspirated. The cells were lysed with a cell lysing solution (cell lysis buffer LCβ; Toyo Ink Mfg.). A luciferase activity of each lysate was measured by a commercially available measuring kit (PicaGene Luminescent kit; Toyo Ink Mfg.) and a measuring apparatus (ML3000 microtiter plate luminometer; Dynatech Laboratories).

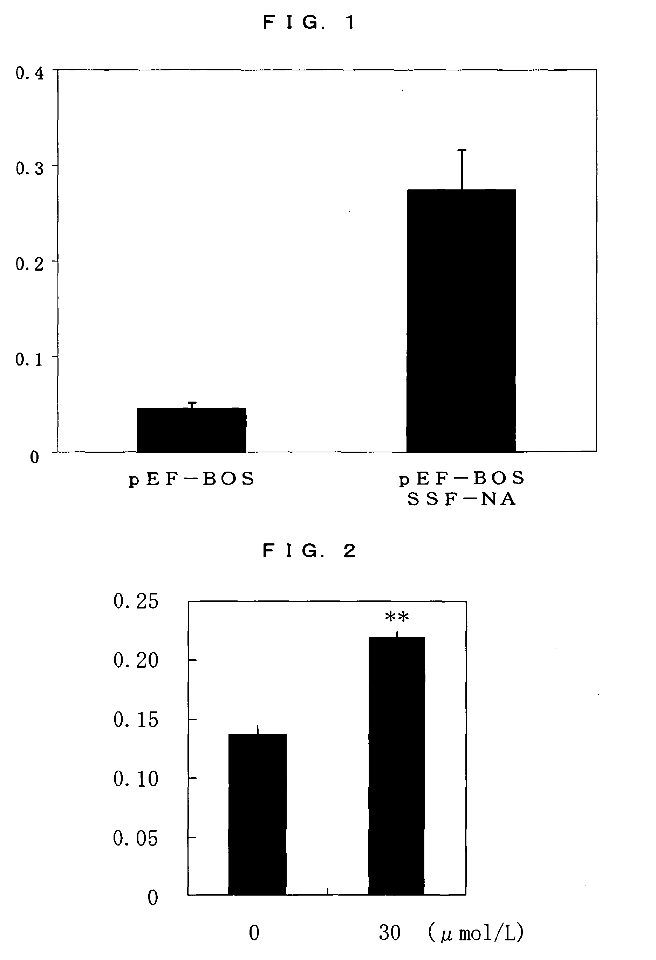

The results (average ± standard error, n = 4) are shown in Figure 1. As shown in Figure 1, the insulin promoter activity in the cell transfected with plasmid pEF-BOS SSF-NA was significantly increased in comparison with that in the cell transfected with plasmid pEF-BOS. The value of Student's t-test was P = 0.0018. It was found that an insulin promoter activity is increased by overexpression (i.e., activation) of the polypeptide consisting of the amino acid sequence of SEQ ID NO: 2.

From the above results, it is considered that an increased insulin promoter activity caused by activating the polypeptide consisting of the amino acid sequence of SEQ ID NO: 2 results in an increase of an insulin biosynthesis and, as a result, an increase of an insulin content. Therefore, it is considered that agonists against the polypeptide consisting of the amino acid sequence of SEQ ID NO: 2 can be used as an agent for preventing and/or treating diabetes, particularly an agent for increasing an insulin content (biosynthesis). The term "agonist" means a substance capable of enhancing a signal of the polypeptide consisting of the amino acid sequence of SEQ ID NO: 2 and enhancing an action of the polypeptide, i.e., a substance activating the polypeptide.

### Example 4: Screening of substances capable of modifying activity of polypeptide consisting of the amino acid sequence of SEQ ID NO: 2, based on change of intracellular cAMP concentration

In the present example, a 293-EBNA cell (Invitrogen), obtained by introducing an EBNA-1 gene of Epstein Barr Virus into a human embryonic kidney derived HEK293 cell, was used as a host cell.

293-EBNA cells (1×10⁴ cells/well) were seeded on 96-well plate coated with collagen, and cultured overnight in a Dulbecco modified Eagles' medium (DMEM) containing 10% fetal calf serum (FCS). Then, a transfection reagent (LIPOFECTAMINE 2000; GIBCO BRL) was used to transfect the cells with 0.01 ng of plasmid pEF-BOS-NA or plasmid pEF-BOS (control vector), together with 5 ng of pCRE-Luc vector (CLONTECH). After the transfection, the cells were cultured for 18-20 hours. Then, test substances diluted with the medium were added, and the cells were incubated at 37°C for 5-6 hours in the presence of 5% CO₂. After the medium was aspirated, the cells were lysed with a cell lysing solution (cell lysis buffer LCβ; Toyo Ink Mfg.). A luciferase activity of each lysate was measured by a commercially available measuring kit (PicaGene Luminescent kit; Toyo Ink Mfg.) and a measuring apparatus (ML3000 microtiter plate luminometer; Dynatech Laboratories).

When an increased value of the reporter activity in the plasmid pEF-BOS-NA-transfected cell treated with a test substance is 1.5 times (preferably 5 times) or more, with respect to that in the plasmid pEF-BOS-transfected cell treated with the test substance, such a test substance can be selected as a compound activating the polypeptide consisting of the amino acid sequence of SEQ ID NO: 2. Alternatively, cells expressing the polypeptide consisting of the amino acid sequence of SEQ ID NO: 2 can be used to measure the activity of a test substance by directly measuring an intracellular cAMP content after treatment with the test substance in accordance with a known method.

The method described in the present example can be used in confirming whether or not compounds selected in Example 5 can activate the polypeptide consisting of the amino acid sequence of SEQ ID NO: 2.

### Example 5: Screening method based on insulin promoter reporter activity

Substances activating the polypeptide consisting of the amino acid sequence of SEQ ID NO: 2 can be screened by introducing the polypeptide consisting of the amino acid sequence of SEQ ID NO: 2 and plasmid InsPro to a cell not expressing the polypeptide. Alternatively, as shown in the present example, compounds can be evaluated by introducing plasmid InsPro to a pancreatic β cell line known to express a mouse derived polypeptide corresponding to the polypeptide consisting of the amino acid sequence of SEQ ID NO: 2 (see Examples 2 and 3 in International Publication WO02/44362). The screening method described in the present example can be used in confirming the action of compounds selected in Example 4 on enhancing the insulin promoter activity.

A transfection reagent (LIPOFECTAMINE 2000; GIBCO BRL, or FuGENE6; Boeringer Mannheim) was used to introduce plasmid InsPro (1-10 ng) prepared in Example 2 to mouse pancreatic β cell line NIT1 cells (4×10⁴ cells). The trasfected cells were seeded on a 96-well plate. A Dulbecco modified Eagles' medium (DMEM) or F-12 medium containing 10% fetal calf serum (FCS) was used as a medium. After the seeding, the cells were cultured for 18-20 hours. Then, test substances diluted with the medium were added, and the cells were incubated at 37°C for 24 hours in the presence of 5% CO₂. After the medium was aspirated, the cells were lysed with a cell lysing solution (cell lysis buffer LCβ; Toyo Ink Mfg.). A luciferase activity of each lysate was measured by a commercially available measuring kit (PicaGene Luminescent kit; Toyo Ink Mfg.) and a measuring apparatus (ML3000 microtiter plate luminometer; Dynatech Laboratories). When a significant increase in the reporter activity by treatment with a test substance is observed with respect to a control (only solvent), it can be judged that such a test substance can activate the insulin promoter activity. Further, where the above procedure is repeated except for using a cell not expressing the polypeptide for a screening tool, as a control cell, instead of the cell for a screening tool, when the insulin promoter reporter activity is not increased in the control cell, it can be judged that such a test substance can activate the polypeptide consisting of the amino acid sequence of SEQ ID NO: 2.

As described above, compounds activating the polypeptide consisting of the amino acid sequence of SEQ ID NO: 2 and increasing the insulin promoter activity can be selected, in accordance with the method described in Example 4 or 5, or the combination thereof.

### Example 6: Screening of substances capable of modifying activity of polypeptide consisting of the amino acid sequence of SEQ ID NO: 2, based on change of intracellular cAMP concentration and insulin promoter reporter activity

Compound screening was carried out by the method described in Example 4 to obtain 2-(pyridine-4-yl)ethyl thiobenzoate (LT-1 Z 0059519 ; LaboTest, hereinafter referred to as compound A). Further, a similar procedure was repeated except that the transfection was carried out after 3 hours from the cell seeding, to obtain 4-{5-[(E)-(1,3-diethyl-5-oxo-2-thioxoimidazolidine-4-ylidene)methyl]-2-furyl}benzoic acid (AN-465/14458032 ; SPECS, hereinafter referred to as compound B), (2Z)-2,3-bis(3,4-dimethoxyphenyl)acrylonitrile (J. Org. Chem., 48, 4222-4232, 1983, hereinafter referred to as compound C), 4-[(E)-2-(3,4-dimethoxyphenyl)vinyl]pyridine (BAS 1550277; ASINEX, hereinafter referred to as compound D), and 5-{[4-(3-methyl-1,2,4-oxadiazole-5-yl)benzyl]thio}-1H-1,2,4-triazole-3-amine (H-066028 ; SCIEXCH, hereinafter referred to as compound E). The compounds A-E were selected as a compound activating the polypeptide consisting of the amino acid sequence of SEQ ID NO: 2. More particularly, each increased value of the reporter activity in the plasmid pEF-BOS-NA-transfected cell treated with each compound (10 µmol/L), with respect to that in the plasmid pEF-BOS-transfected cell treated therewith, was 3 times or more (compound A, D, or E) or 5 times or more (compound B or C).

Next, compounds A-E were evaluated in a manner similar to that described in Example 5. More particularly, a 96-well plate coated with collagen was used as the 96-well plate, and DMEM was used as the medium. Further, the treatment with compound A was carried out for 31 hours, and that with compound B-E was carried out for 24 hours. The results (average ± standard error, n = 6) are shown in Figures 2 and 3. The increased value of the reporter activity in each group treated with compound A (30 µmol/L) or compound B-E (10 µmol/L) was significantly 1.5 times or more with respect to that in a control group (i.e., compound concentration = 0 µmol/L). The symbol "**" in Figures 2 and 3 denotes that the significant difference with respect to the control group (i.e., without compounds A-E) was p<0.01 (Student's t-test).

As described above, it was found that compounds A-E activating the polypeptide consisting of the amino acid sequence of SEQ ID NO: 2 can increase the insulin promoter activity, i.e., can promote the insulin production and increase the insulin content.

### Referential Example: Glucose tolerance tests for SD rats and GK rats by one oral dose

SD rats (4 weeks old; CLEA JAPAN) were made to fast overnight, and 2 g/kg of glucose was orally administered. Compound A (100 mg/kg) had been intraperitoneally administered 5 minutes before the glucose administration. An appropriate amount of blood was taken at 0 minute, 30 minutes, 60 minutes, and 120 minutes after the glucose administration, and used for the measurement of a blood glucose level and a concentration of plasma insulin.

For measuring the blood glucose level, a supernatant obtained by mixing blood and 0.33 mol/L perchloric acid (blood:0.33 mol/L perchloric acid = 1:10) and centrifuging the mixture (3000 x g, 10 minutes, 4°C) was used. For measuring the concentration of plasma insulin, a supernatant obtained by centrifuging blood (3000 x g, 10 minutes, 4°C) was used. Further, Glucose C test Wako (Wako) was used in the measurement of the blood glucose level, and a rat insulin assay system (Amersham) was used in the measurement of the concentration of plasma insulin.

The results are shown in Figures 4 and 5. Figure 4 illustrates a time course of the concentration of plasma insulin (unit = ng/mL) after the oral administration of glucose, and Figure 5 illustrates a time course of the blood glucose level (unit = mg/dL) after the oral administration of glucose. The mark "*" in Figures 4 and 5 denotes that a significant difference from the group to which compound A had not been administered was p<0.05 (Student's t-test).

As shown in Figure 4, a significant increase of the concentration of plasma insulin was observed at 30 minutes after glucose administration, by administration of 100 mg/kg of compound A. Further, an increase of the blood glucose level by the glucose administration was significantly suppressed at 30 minutes after glucose administration, in the group to which 100 mg/kg of compound A had been administered.

Therefore, it was confirmed that, in the SD rats to which glucose was administered, compound A exhibited a function to increase the amount of insulin in plasma, and a function to reduce the blood glucose level.

Then, a glucose tolerance test for GK (Goto-Kakizaki) rats (type II diabetes models with incomplete insulin secretion; 7 weeks old; Charles River Japan) by one oral dose was carried out. The GK rat line was established by selectively mating wistar rats in accordance with an index of a poor tolerance in an oral glucose tolerance test, by Yoshio Goto, et al., School of Medicine, Tohoku University, in 1975.

The procedures of the glucose tolerance test for SD rats were repeated except that compound A was orally administered.

Figure 6 illustrates a time course of the blood glucose level (unit = mg/dL) after the oral administration of glucose. In Figure 6, the mark "*" denotes that a significant difference from the group to which compound A had not been administered was p<0.05 (Student's t-test), and the mark "**" denotes that the significant difference as above was p<0.01.

As shown in Figure 6, an increase of the blood glucose level by the glucose administration was significantly suppressed at 30 and 60 minutes after glucose administration, by administration of 100 mg/kg of compound A, and therefore, the utility of compound A was confirmed in the diabetes model rat.

### INDUSTRIAL APPLICABILITY

According to the screening tool or screening method of the present invention, an agent for increasing insulin content, which is capable of promoting insulin production, can be screened. The agent for increasing insulin content is useful in preventing and/or treating diabetes.

### FREE TEXT IN SEQUENCE LISTING

Each of the base sequences of SEQ ID NOS: 5 and 6 is an artificially synthesized primer sequence.

Although the present invention has been described with reference to specific embodiments, various changes and modifications obvious to those skilled in the art are possible without departing from the scope of the appended claims.

## Claims

1. A screening tool for an agent for promoting insulin production and/or an agent for increasing insulin content, wherein the tool is a polypeptide comprising (1) the amino acid sequence of SEQ ID NO: 2 or 4 or (2) an amino acid sequence in which 1 to 15 amino acids are deleted, substituted, and/or inserted in the amino acid sequence of SEQ ID NO: 2 or 4, and exhibiting an activity of promoting insulin production by activation.

2. A screening tool for an agent for promoting insulin production and/or an agent for increasing insulin content, wherein the tool is a polypeptide comprising the amino acid sequence of SEQ ID NO: 2 or 4 and exhibiting an activity of promoting insulin production by activation.

3. A screening tool for an agent for promoting insulin production and/or an agent for increasing insulin content, wherein the tool is a polypeptide consisting of an amino acid sequence having an 80% or more homology with that of SEQ ID NO: 2 or 4, and exhibiting an activity of promoting insulin production by activation.

4. A screening tool for an agent for promoting insulin production and/or an agent for increasing insulin content, wherein the tool is a polypeptide consisting of the amino acid sequence of SEQ ID NO: 2 or 4.

5. A screening tool for an agent for promoting insulin production and/or an agent for increasing insulin content, wherein the tool is a cell expressing the polypeptide according to any one of claims 1 to 4.

6. Use of the polypeptide according to any one of claims 1 to 4 or the cell according to claim 5 for screening an agent for promoting insulin production and/or an agent for increasing insulin content.

7. A method for screening an agent for promoting insulin production and/or an agent for increasing insulin content, comprising the steps of:
bringing the cell according to claim 5 or a cell membrane thereof into contact with a substance to be tested, and
analyzing whether or not the polypeptide according to any one of claims 1 to 4 is activated.

8. A process for manufacturing a pharmaceutical composition for promoting insulin production and/or increasing insulin content, comprising the steps of:
bringing the cell according to claim 5 or a cell membrane thereof into contact with a substance to be tested,
analyzing whether or not the polypeptide according to any one of claims 1 to 4 is activated, and
preparing a medicament containing the substance.

9. An agent for promoting insulin production and/or an agent for increasing insulin content, comprising as an active ingredient a substance activating the polypeptide according to any one of claims 1 to 4.

10. A method for promoting insulin production and/or increasing insulin content, comprising administering to a subject a substance activating the polypeptide according to any one of claims 1 to 4.

11. Use of a substance activating the polypeptide according to any one of claims 1 to 4, in the manufacture of a pharmaceutical composition for promoting insulin production and/or increasing insulin content.
